(19) Europäisches Patentamt
European Patent Office
Office européen des brevets

(11) **EP 3 765 081 B1**

(12) **EUROPEAN PATENT SPECIFICATION**

(45) Date of publication and mention of the grant of the patent:
**22.11.2023 Bulletin 2023/47**

(21) Application number: **19766828.8**

(22) Date of filing: **12.03.2019**

(51) International Patent Classification (IPC):
*A61K 41/00* (2020.01)  *A61K 36/28* (2006.01)
*A61P 17/02* (2006.01)  *A61P 31/00* (2006.01)

(52) Cooperative Patent Classification (CPC):
(C-Sets available)
**A61P 17/02; A61K 31/5415; A61K 36/28;
A61K 41/0057; A61K 45/06; A61P 31/00;
Y02A 50/30**                                    (Cont.)

(86) International application number:
**PCT/CA2019/050298**

(87) International publication number:
**WO 2019/173907 (19.09.2019 Gazette 2019/38)**

(54) **ANTIMICROBIAL PHOTOSENSITIZER COMPOSITION**

ANTIMIKROBIELLE FOTOSENSIBILISATORZUSAMMENSETZUNG

COMPOSITION PHOTOSENSIBLE ANTIMICROBIENNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2018 US 201862641685 P**

(43) Date of publication of application:
**20.01.2021 Bulletin 2021/03**

(73) Proprietor: **Toefx Inc.
Hamilton, Ontario L8P 0A1 (CA)**

(72) Inventor: **VAN-HAM, Irit Itzhaki
Vaughan, Ontario L4J 9G9 (CA)**

(74) Representative: **Bosch Jehle
Patentanwaltsgesellschaft mbH
Flüggenstraße 13
80639 München (DE)**

(56) References cited:
EP-A1- 0 006 060        WO-A1-2017/203359
WO-A2-2012/090194       US-A1- 2009 233 914
US-A1- 2009 234 270

- **MOUHAJIR F ET AL: "Multiple antiviral activities of endemic medicinal plants used by Berber peoples of Morocco", PHARMACEUTICAL BIOLOGY, SWETS AND ZEITLINGER, LISSE, NL, vol. 39, no. 5, 1 October 2001 (2001-10-01), pages 364-374, XP009152581, ISSN: 1388-0209**
- **LUDMILA M. BALTAZAR ET AL: "Antimicrobial photodynamic therapy: an effective alternative approach to control fungal infections", FRONTIERS IN MICROBIOLOGY, vol. 6, 13 March 2015 (2015-03-13), page 202, XP055242757, DOI: 10.3389/fmicb.2015.00202**
- **NJOKI LOISE M. ET AL: "Effects of Medicinal Plant Extracts and Photosensitization on Aflatoxin Producing Aspergillus flavus (Raper and Fennell)", INTERNATIONAL JOURNAL OF MICROBIOLOGY, vol. 2017, 1 January 2017 (2017-01-01), pages 1-9, XP055812760, ISSN: 1687-918X, DOI: 10.1155/2017/5273893 Retrieved from the Internet: URL:https://www.ncbi.nlm.nih.gov/pmc/artic les/PMC5433414/pdf/IJMICRO2017-5273893.pdf >**

- AL-FAWWAZ ABDULLAH T. ET AL: "Antibacterial And Antifungal Effect Of Some Natural Extracts And Their Potential Use As Photosensitizers", EUROPEAN SCIENTIFIC JOURNAL, ESJ, vol. 12, no. 6, 29 February 2016 (2016-02-29), page 147, XP055812763, ISSN: 1857-7881, DOI: 10.19044/esj.2016.v12n6p147 Retrieved from the Internet: URL:http://eujournal.org/index.php/esj/art icle/viewFile/7054/6815>
- BERHAIL BOUDOUDA et al.: "Antibacterial activity and chemical composition of essential oils of Inula viscosa (L.) Ait. (Asteraceae) from Constantine, Algeria", Der Pharmacia Lettre, vol. 4, no. 6, 2012, pages 1878-1882, XP55635828,
- MAOZ et al.: "Effect of Inula viscosa extract on chitin synthesis in dermatophytes and Candida albicans", Journal of Ethnopharmacology, vol. 71, 2000, pages 479-482, XP028143266,
- TALIB et al.: "Antiproliferative, antimicrobial and apoptosis inducing effects of compounds isolated from Inula viscosa", Molecules, vol. 17, 2012, pages 3291-3303, XP055299252, DOI: doi:10.3390/molecules17033291
- MAHMOUDI et al.: "Comprehensive phytochemical analysis, antioxidant and antifungal activities of Inula viscosa aiton leaves", Journal of Food Chemistry, vol. 36, 2016, pages 77-88, XP055465761, DOI: doi:10.1111/jfs.12215
- GOKBULUT et al.: "Antioxidant and antimicrobial activities, and phenolic compounds of selected Inula species from Turkey", Natural Product Communications, vol. 8, no. 4, 2013, pages 475-478, XP55635832,
- LARDI et al.: "The antibacterial effect of two medicinal plants Inula viscosa, Anacyclus valentiunus (Asteraceae) and their synergistic interaction with antibiotic drugs", Journal of Fundamental and Applied Sciences, vol. 8, no. 2, 2016, pages 244-255, XP55635837,
- DANINO et al.: "Antioxidant activity of 1,3-dicaffeoylquinic acid isolated from Inula viscosa", Food Research International, vol. 42, 2009, pages 1273-1280, XP026458621,

(52) Cooperative Patent Classification (CPC): (Cont.)

C-Sets
A61K 31/5415, A61K 2300/00;
A61K 36/28, A61K 2300/00

**Description**

FIELD OF THE INVENTION

The present invention pertains to a composition for photodynamic therapy. In particular, the photodynamic therapy can be used for the treatment of yeast, fungal, bacterial, and viral skin, wart, nail, and topical infections.

BACKGROUND

Photodynamic therapy (PDT), also known as photochemotherapy or photobiomodulation or photodisinfection, is a therapeutic method that uses light and a photosensitizing agent in conjunction with oxygen to elicit cell death or phototoxicity. PDT has historically been demonstrated to be useful in killing various microorganisms when the appropriate dye and light are combined, however it is only in relatively recent times that PDT has been studied as a treatment for various types of localized infections including fungus, bacteria, and other pathogens. In particular, PDT has been used for locally disinfecting or sterilizing a hard or soft tissue site by topically applying a photosensitizing agent to the site, and then irradiating the site with light at a wavelength that is absorbed by the photosensitizing agent so as to destroy microbes at the site. Photodynamic therapy has been used in medicine to kill bacteria (including *Staphylococcus aureus, Escherichia coli* and *Pseudomonas aeruginosa*), fungi (including *Trichophyton rubrum* and *Candida sp.*), viruses (including human immunodeficiency virus, methicillin resistant staphylococcus aureus, and Vancomycin-resistant enterococci), and to treat certain cancers (including non-melanoma skin cancer, esophageal cancer and non-small cell lung cancer), wet age-related macular degeneration, acne, and psoriasis. Photodynamic treatment has also been used for disinfection in dental applications.

Photosensitizers, when excited with light, assist to produce radicals and/or reactive oxygen. Although photosensitizers may have no antimicrobial or antifungal activity at low concentrations or without light, when irradiated with light of an appropriate excitation wavelength they are able to kill a wide range of microbes by producing forms of radicals and/or reactive oxygen at the site of microbial or fungal growth. In the presence of oxygen, the light exposure of photosensitizer and energy transfer can lead to two main photochemical reactions (Type I and Type II reaction) and generation singlet oxygen $^1O_2$ and a reactive oxygen species (ROS). The antimicrobial effect is thought to be due to a physical disruption of the microbial cell membrane through oxidative reactions, particularly initiated by singlet oxygen produced upon irradiation in the presence of the photosensitizer.

Toenail fungus, also known as *tinea unguium* (ringworm of the nails), or onychomycosis, is a fungal infection of the nail, affecting fingernails and more commonly toenails. This condition is caused primarily by members of a group of parasitic fungi known as *Trichophyton rubrum* (*T. rubrum*) or *Trichophyton mentagrophytes.* Onychomycosis causes the nails to thicken, discolour, disfigure, and split. Onychomycosis accounts for about half of all nail disorders and is the most common nail disease in adults, constituting about half of all nail abnormalities. Onychomycosis is found in about 10% of the general population, with people in colder climates such as Northern Europe and Canada having a greater prevalence than those in tropical and sub-tropical regions. In the adult population, prevalence increases with age. For example, globally, about 20% of individuals 60 years and older, and 50% of individuals over 70 years have onychomycosis. Furthermore, in recent years, the incidence of onychomycosis has been increasing, primarily due to an increasing population of patients with diabetes, peripheral vascular diseases, immunologic disorders, and an aging population. Onychomycosis is caused by three main classes of organisms: dermatophytes (fungi that infect hair, skin, and nails and feed on nail tissue), yeasts, and non-dermatophyte molds. The most common symptoms of a fungal nail infection are the nail becoming thickened and discoloured, turning white, black, yellow or green. As the infection progresses the nail can become brittle, with pieces breaking off or coming away from the toe or finger completely. If left untreated, the skin underneath and around the nail can become inflamed and painful. There may also be white or yellow patches on the nail bed or scaly skin next to the nail, and a foul smell. People with onychomycosis may also experience embarrassment due to the appearance of the nail, especially with an infected fingernail. The causative pathogens of onychomycosis are predominantly in the fungus kingdom and include dermatophytes, Candida (yeasts), and non-dermatophytic molds. The current standard of care for onychomycosis is an oral antifungal agent, such as either terbinafine or litraconazole. However, relatively small amounts of systemic oral drugs can reach the target nail site and the cure rate using these treatments is less than 10%. Often, treatment duration may extend to one year, which can lead to significant systemic side effects such as liver and cardiac damage. In addition, physical treatments such as surgery and laser treatment have been tested, but the treatments are expensive, the cure rate poor, and mild to severe side effects may occur.

United States patent applications US 2009/0233914 A1 and US 2009/0234270 A1 to Loebel describe a composition for treating fungal infections, particularly onychomycosis, comprising a photosensitizer, an effective amount of antifungal agent, and a pharmaceutically acceptable delivery system. Loebel describes a variety of composition components, photosensitizers and antimicrobial compounds, presenting data on in vitro testing. The photosensitizer composition used by Loebel is disclosed as comprising 0.01 % w/v of methylene blue. Broad exemplary protocols for nail treatment are described without details for treatment compositions, photodynamic therapy conditions and therapy regimens, and outcomes for treatment of onychomycosis.

Document WO 2012/090194 A2 relates to compositions, methods and kits for treating topical disorders, some of which are associated with a viral infections, the composition comprising at least one of a plant extract comprising at least one of tomentosin and inuviscolide, tomentosin, inuviscolide, a plant cell culture extract comprising at least one of tomentosin and inuviscolide and an Inula viscosa extract and a pharmaceutically acceptable carrier.

The *Inula Viscosa* is a sturdy perennial shrub that grows in the wild around the Mediterranean Basin. It has been used for years in folk medicine for its anti-inflammatory, antipyretic, antiseptic, arthritis and wound healing. The leaves of the plant can be boiled to create a medicinal tincture, or they can be extracted to create an oleoresin.

There remains a need for an efficacious, efficient, safe with limited compliance issues, photodynamic therapy which can be used for the treatment of yeast, fungal, viral, wart and bacterial skin and topical infections.

This background information is provided for the purpose of making known information believed by the applicant to be of possible relevance to the present invention. No admission is necessarily intended, nor should be construed, that any of the preceding information constitutes prior art against the present invention.

SUMMARY OF THE INVENTION

An object of the present invention is to provide a photodynamic anti-microbial therapy which can be used for the treatment of yeast, fungal, viruses, wart, and bacterial skin, nail and topical infections.

The invention is defined by the independent claim. Embodiments of the invention are indicated in the dependent claims.

In an embodiment, the *inula viscosa* extract acts as both an antimicrobial and a photosensitizer in the composition.

In another embodiment, the composition further comprises an additional photosensitizer. In an embodiment, the additional photosensitizer is a plant-derived photosensitizer. In another embodiment, the additional photosensitizer is one of hypericin, chlorophyll, curcumin, methylene blue, and a combination thereof. In another embodiment of the composition, the additional photosensitizer one of a porphyrin, phthalocyanine, phenothiazinium, benzoporphyrins, haematoporphyrin, pyrrole, tetrapyrrolic compound, pyrrolic macrocycle, porfimer, and 5-aminolevulinic acid.

In another embodiment, the composition comprises an additional antimicrobial selected from a cinnamodial sterol, furanone, quinine, resorcinol, or terpenoid.

In another embodiment of the composition, the *inula viscosa* extract is present in the composition in 0.1%-10% v/v. In another embodiment, the composition further comprises propolis.

In another embodiment, the composition further comprises a penetration enhancer.

In another embodiment, the composition further comprises a chelator.

In another embodiment, the composition further comprises a preservative.

In another embodiment of the composition, the pharmaceutically acceptable vehicle comprises: glycerin in 10-50 v/v%; isopropanol, ethanol, or a mixture thereof in 5-35 v/v%; and a buffer or water in 25-75 v/v%.

In another embodiment, the composition further comprises a synthetic antifungal.

In another embodiment, the composition further comprises an anti-inflammatory compound.

DETAILED DESCRIPTION OF THE INVENTION

Unless defined otherwise, all technical and scientific terms used herein have the same meaning as commonly understood by one of ordinary skill in the art to which this invention belongs.

As used in the specification and claims, the singular forms "a", "an" and "the" include plural references unless the context clearly dictates otherwise.

The term "comprising" as used herein will be understood to mean that the list following is non-exhaustive and may or may not include any other additional suitable items, for example one or more further feature(s), component(s) and/or element(s) as appropriate.

The terms "photosensitizer," "photosensitizing agent" and "phototherapy agent" as used herein interchangeably to refer to a chemical species or compound which initiates a cytotoxic chemical reaction or produces a reactive oxygen species (ROS) when exposed to a specific wavelength of light. Photosensitizers are commonly formulated as part of a photosensitizer composition for use in PDT.

The term "photodynamic therapy" as used herein refers to a treatment method of treating a tissue site by topically applying a photosensitizer to the site, and then irradiating the site with light at a wavelength that is absorbed by the photosensitizer compound so as to generate a reactive oxygen species at the location where the light source excites the photosensitizer.

The term "light" as used herein refers to an electromagnetic spectrum emission at any wavelength. In the present application, the light contribution to the photodynamic therapy is of an appropriate wavelength capable of being absorbed by a photosensitizer or photosensitizing composition.

Phototherapy, also known as photobiomodulation, involves the application of light to stimulate cellular processes and enhance healing. Wavelengths of light used with photosensitizers include those selected from the continuous electromagnetic spectrum such as ultraviolet ("UV"), visible, and infrared (near, mid and far), etc. The light may be produced by any suitable light emitting device such as lasers, light emitting diodes ("LEDs"), incandescent sources, fluorescent sources, or the like.

The present invention is directed to a composition for the treatment of dermatological microbial infections. The antimicrobial composition of the present invention comprises *inula viscosa* extract, which acts a photosensitizer. The present compositions can be used in combination with photodynamic therapy as a natural and effective treatment for antimicrobial infections, and more particularly for topical infections caused by fungus, bacteria, virus, warts, and dermatophytes. These topical infections also include skin infections and nail infections such as onychomycosis. As the present compositions are agnostic with regard to the target and produce activated oxygen species at the site of infection and localized photodynamic treatment, the present methods and compositions can be used to disinfect and treat infections caused by a variety of microbes at light-accessible locations on the body. Photodynamic therapy using the present light-sensitive agents and compositions in combination with specific wavelengths of light creates local toxicity to targeted diseased cells and microorganisms, known as phototoxicity. Photodynamic therapy further in combination with an antimicrobial agent can provide a synergistic effect in that photodynamic therapy delivers immediate killing of microbes and disinfects the locus while the antifungal agent provides long term killing of fungi at the locus. Compared to systemic chemotherapy, the present composition and therapy is of short duration, easy to apply and administer, and is more directed, providing a more targeted treatment.

Photosensitizer

Photodynamic therapy (PDT) induces the formation of Reactive Oxygen Species (ROS). The anti-microbial photodynamic effect of the therapy depends on physical and chemical parameters of the photosensitizer, e.g., absorption peak ($k_{max}$), intensity of absorption ($e_{max}$), and quantum yield for singlet oxygen. In Type I photodynamic therapy, the activated photosensitizer transfers an electron to form a free radical, which forms a highly reactive oxygen species (ROS). The radical interacts with oxygen to produce superoxide anion radical ($\overset{\cdot}{O}{\cdot}^{-}_{2}$) which can cause oxidative damage to cell structures, and is therefore cytotoxic. In contrast, in Type II photodynamic therapy, activated photosensitizer transfers its energy directly to oxygen to form singlet oxygen ($^{1}O_{2}$) which is a highly reactive oxygen species (ROS). The reactive oxygen species generated oxidizes various cellular substrates (ie. cell membrane, DNA, mitochondria, lysosomes) and is thereby cytotoxic. Singlet oxygen has a very short lifetime (~3$\mu$s) and must be generated in close proximity to cells to produce cytotoxic effects. In the case of Methylene Blue, it is hypothesized that a Type II reaction occurs.

Photosensitizers generally have a stable electronic configuration, which is in a singlet state in their lowest or ground energy level. Following absorption of a photon of light of specific wavelength, the photosensitizer molecule is promoted to an excited state, which is also a singlet state and is short lived with a half-life. The photosensitizer can then return to the ground state by emitting a photon as light energy or by internal conversion with energy lost as heat. Microorganisms that are killed by singlet oxygen include viruses, bacteria, protozoa, and fungi. The action of singlet and superoxide radical is locally limited due to the very short diffusion path of the reactive oxygen species. Due to the short half-life of the ROS, the reaction stops immediately after the light is switched off.

Molecules possessing photosensitizing capability are typically rigid planar structures possessing a high degree of conjugation. Synthetic photosensitizer classes which can be used include but are not limited to porphyrins, phthalocyanines, and phenothiaziniums. Other substances, such as 5-aminolevulinic acid (ALA) and naturally occurring substances such as curcumin, have also been used as photosensitizers. ALA is not intrinsically photodynamically active, but irradiation of cells containing ALA produces a range of endogenous photosensitizers that generate reactive oxygen species (ROS), which damage mitochondria and plasma membranes (Harris and Pierpoint, Med. Res. Rev. 32, pp.1292-1327, 2012). Phthalocyanines have a light absorption in the range of 630-720 nm and are similar to porphyrin compounds (Calzavara-Pinton et al., J. Photochem. Photobiol. 8 Biol. 78, pp.1-6, 2012; Sekkat et al., Molecules 17, pp. 98-144, 2012), however they are strongly hydrophobic, a characteristic that is usually balanced by modifications in its chemical structure to improve water solubility (Mantareva et al., Eur. J. Med. Chem. 46, 4430-4440 2011; Sekkat et al., Molecules 17, pp. 98-144, 2012). Phenothiaziniums have simple tricyclic planar structures, and are typically cationic in nature. Phenothiaziniums suitable for use in PDT include but are not limited to methylene blue (MB) and toluidine blue (TBO). Both are efficient producers of singlet oxygen and have a maximum absorption wavelength in water of 656nm for MB and 625 nm for TBO, respectively. Methylene blue (MB) has a maximum wavelength of absorption in the range of 600-660 nm (Calzavara-Pinton et al., J. Photochem. Photobiol. 8 Biol. 78, pp.1-6, 2012). Porphyrins are heterocyclic macrocycles derived from four pyrrole-like subunits interconnected via their carbon atoms via methine bridges. Porphyrins compound suitable for use in PDT include but are not limited to porfimer sodium (tumoricidal capacity), benzoporphyrin derivatives (tumoricidal capacity), and cationic phenothiazinium dyes (antimicrobial. Porphyrin dyes have a absorption in the 400-650 nm range and can cause alterations at cell membranes, allowing the penetration of the photosensitizer into the cell with consequent damage to intracellular targets (Cormick et al., Eur. J. Med. Chem. 44, 1592-1599, 2009; Calzavara-Pinton et al., J. Photochem. Photobiol. 8 Biol. 78, pp.1-6, 2012).

Hypericin, a naturally occurring pigment, is found in certain species of plants from the genus Hypericum, the most common of which is Saint John's Wort (Hypericum perforatum). Hypericum perforatum comprises a variety of active compounds including hypericin, pseudohypericin, flavonoids, biflavones, hyperforin, tannins, procyanidines, xanthones, chloregenic acid, caffeoylquinic, and p-coumaroylquinic acids. (Chitroda et al., International Journal of Scientific Study, August 2014, Vol 2, Issue 5). Naphthodianthrone compounds present in *Hypericum* sp. (hypericin), including *Hypericum perforatum* L. and *Hypericum erectum* C.P. Thunberg ex A. Murray (Hypericaceae) and buckwheat plants (fagopyrin) have also been shown to a photosensitizing effect (Sytar et al. Pharmaceutical Biology Vol. 54, Iss. 12, 2016). In addition, naphthodianthrones like hypericin, hypericin + S and fagopyrin are natural photosensitizer considered for the new generation of photodynamic therapy drugs. It was found that hypericin had a high phototoxicity to *Staphylococcus aureus, Enterococcus faecalis* and *Escherichia coli* at extremely low drug concentrations (Kashef et al. Photodiagnosis Photodyn Ther. 10, pp.150-155, 2013). It was found also that hypericin is inducible by pathogen/herbivore attack or if it could play a role in plant defence under plant pathogens *Phytophthora capsici* and *Diploceras hypericinum* (Çirak et al. Plant Protect Sci. 41, pp.109-114, 2005). Extracts from *Hypericum* sp. have also been tested opportunistic against pathogenic yeasts and filamentous fungi, including dermatophytes (Fenner et al., Phytomedicine, Mar 12(3), pp.236-40, 2005).

Curcumin is a small-molecular-weight yellow compound that is isolated from the rhizomes of *Curcuma longa* L. and is one of the components of the commonly used spice turmeric. Curcumin (diferuloylmethane) is a a diarylheptanoid belonging to the group of curcuminoids, which are natural phenols produced by some plants, including tumeric. Curcumin has been associated with antioxidant, anti-inflammatory, anticancer, antiviral, and antibacterial activities. (Prasad, S. et al., Cancer Res Treat. Jan 2014, 46(1), pp. 2-18). Some studies have shown that curcumin can have antifungal activity against *Candida* spp. and also the capability to inhibit fluconazole resistance and to increase the fungicidal effects of amphotericin B in *Candida* isolates. Additionally, recent investigations showed that the antifungal effects of curcumin may be enhanced by combination with light, especially in the blue spectral region. Due to the superficial penetration depth of blue light into tissues, curcumin may be used as a suitable photosensitizer for treatment of localized superficial infections such as oral candidiasis. Low curcumin concentrations have been found to be highly effective for photoinactivating C. *albicans,* in planktonic and biofilm form. Further, it has been found that therapy with curcumin was more effective in inactivating a yeast cell than a macrophage cell line, suggesting a certain specificity of curcumin-mediated PDT. Curcumin also has a light absorption in the 408-434 nm range and is a known photosensitizer (Dovigo et al., Photochem. Photobiol. 87, pp.895-903, 2013) PDT with curcumin generates high levels of ROS that cause cell death by apoptosis (Sharma et al., Biosci. Rep. 30, pp.391-404, 2010).

Chlorophylls are naturally occurring substituted chlorin derivatives. Chlorophyll A and Chlorophyll B occur in all green plants and are the most common natural chlorophylls. In chlorophylls the longest wavelength absorption band shifts to 650 to 690 nm range and increases several fold in height, which are factors highly desirable to photosensitizers. An additional advantage of using Chlorophyll A is economic, in that Chlorophyll A can be isolated in large amounts from plants or algae. *Spirulina* algae, which contains only Chlorophyll A, is also commercially available in the form of freeze-dried powder. Natural chlorophylls and derivatives are also biosynthetically related to protoporphyrin IX, which is present in higher organisms. Consequently, the biocompatibility of natural related photosensitizers is expected, mainly in terms of pharmacokinetic clearance. Chlorophylls can also be used as starting materials for the synthesis of functionalized chlorins, since some pharmacophoric groups can be introduced to improve the photodynamic activity. Chlorophylls constitute useful templates for use as PSs considering their photophysical and photobiological proprieties, and their high level of functionalization that permits chemical transformations. In general, chlorophyll derivatives are good candidates for photodynamic inactivation because they have a better selectivity in microorganisms compared to mammalian cells, especially with low incubation times. It has been found that *inula viscosa* acts as a photosensitizer in the present composition and phototherapy, and that compositions comprising *inula viscosa* extract provide improved efficacy compared to use of the same composition in the absence of phototherapy. Components in the *inula viscosa* such as chlorophyll could be serving as natural photosensitizers at the same time as the same or other components in the *inula viscosa* extract act as effective antimicrobials, and in particular as antifungals.

In addition *inula viscosa* extract, other additional photosensitizers can be used in the present composition such as but not limited to toluidine blue O, crystal violet, methylene blue, azure dyes, porphyrins, benzoporphyrins, haematoporphyrins, phthalocyanines, pyrroles, tetrapyrrolic compounds, pyrrolic macrocycles, phenothiazinium dyes, porfimers, 5-aminolevulinic acid (ALA) and derivatives thereof.

Antimicrobial

Antimicrobials are chemical species, compositions, or compounds that kill, arrest, or stop growth of microorganisms. Antimicrobials can be active against a variety of infection-causing microbes including but not limited to bacteria (antibacterials) and fungi (antifungals). An antifungal agent, or fungicide, is a biocidal chemical compound, composition, group of compounds, or biological organism used to kill or inhibit growth of fungi, fungal structures, or fungal spores. A wide variety of natural antifungals are known, including compounds and metabolites from marine invertebrates, plants, and various other natural sources. Natural antifungal compounds come in a wide variety of structural classes ranging from cinnamodial sterols, furanones, quinines, resorcinols, and terpenoids. The antifungal activity of compounds derived from natural sources is normally achieved within a few active ingredients in the source and normally in safer with fewer side effects in comparison to synthetic compounds.

Natural extracts can comprise a multitude of chemical species and bioactive compounds which can work independently or synergistically. Propolis, a resinous mixture that honey bees produce by mixing saliva and beeswax with exudate gathered from tree buds, sap flows, or other botanical sources, has been found to have antibacterial, antiviral, antifungal, antimitogenic, anti-inflammatory, and immunomodulatory properties. Propolis is the third most important component of bee products and composed mainly of resin (50%), wax (30%), essential oils (10%), pollen (5%), and other organic compounds (5%) . Phenolic compounds, esters, flavonoids, terpenes, beta-steroids, aromatic aldehydes, and alcohols are the important organic compounds present in propolis. Twelve different flavonoids, namely, pinocembrin, acacetin, chrysin, rutin, luteolin, kaempferol, apigenin, myricetin, catechin, naringenin, galangin, and quercetin; two phenolic acids, caffeic acid and cinnamic acid; and one stilbene derivative called resveratrol have been detected in propolis extracts by capillary zone electrophoresis A few enzymes, such as succinic dehydrogenase, glucose-6-phosphatase, adenosine triphosphatase, and acid phosphatase, are also present in propolis.

*Inula viscosa* is a perennial shrub native to the Mediterranean region and has been found to have anti-inflammatory, analgesic and antimicrobial properties. *Inula viscosa* extract contains inuviscolide which is the main anti-inflammatory sesquiterpenoid from *inula viscosa,* and may act by interfering with leukotriene synthesis and PLA2-induced mastocyte release of inflammatory mediators (A *Mechanistic Approach* to *the In Vivo Anti-inflammatory Activity of Sesquiterpenoid Compounds Isolated from Inula viscosa, 2001).* It also contains flavonoids and sesquiterpene which contributes to antimicrobial activity. The antimicrobial impact of *inula viscosa* has been observed and is summarized in Table 1:

Table 1: *Inula Viscosa* Antimicrobial Activity Summary

| Microorganisms | Results | Date of report / publication |
|---|---|---|
| Pseudomonas aeruginosa | Inhibition zone 15-21mm, MIC 20ug/ml | Berhail Boudouda H, Der Pharmacia Lettre 4(6): pp.1863-1867,2012 |
| Echerichia coli | Inhibition zone 13-20mm, MIC 40-80ug/ml 156 ug/ml | Berhail Boudouda H, Der Pharmacia Lettre 4(6): pp.1863-1867,2012 Khadija, 2013 |
| Staphylococcus aureus | Inhibition zone 9-11mm, MIC 80ug/ml Inhibition zone 11mm, MIC 6.25 mg/ml | Berhail Boudouda H, Der Pharmacia Lettre 4(6): pp.1863-1867,2012 Sawsam et al, European Journal of Medicinal Plants 3: 3, pp.394 - 404, 2013 |
| Enterobacter gergovia | *Results* of E. aerogenes, *Inhibition zone 16.5-18mm, MIC 20-80ug/ml* | Berhail Boudouda H, Der Pharmacia Lettre 4(6): pp.1863-1867,2012 |
| Candida albicans | *I. viscosa* extract inhibited the growth of dermatophytes and *C. albicans* | Maoz M, Neeman, J Ethnopharmacol.; 71(3): pp. 479-82, Aug 2000 |
| Aspergillus brasiliensis | *I. viscosa* inhibit the colony growth of the fungi in a dose dependent manner | Mamoci E *et al. Molecules.* 16(3):2609-25, December 2011 |
| Propionibacterium acnes- | MIC 2.5mg/ml | Bassam I. *et al. Afr. J. Pharm. Pharmacol,* Vol. 7(29), pp. 2087-2099, 8 August, 2013 |

Lamprini Karygianni et *al. (BioMed Research International,* Volume 2014, 2014) from the Department of Operative Dentistry and Periodontology, University of Freiburg Germany studied the efficiency of the *inula viscosa* extract against ten bacteria and one *Candida albicans* strain. The extraction protocols were conducted according to established experimental procedures. Two antimicrobial assays, a minimum inhibitory concentration (MIC) assay and a minimum bactericidal concentration (MBC) assay, were applied. The screened extract was found to be active against each of the tested microorganisms.

Stabilization of photosensitizers in a vehicle composition to maintain optimal concentrations of photosensitive species assist in concentrating photoactive species at the site of photodynamic therapy. In an example, methylene blue (MB) is most photosensitive in its monomer form, however prone to dimerization and polymerization in solution. MB is a cationic dye that efficiently absorbs in the red wavelength (around 664 nm). MB forms dimers or higher aggregates in solution upon increasing dye concentration. Dimerization increases at higher ionic strength and may also change in the presence of charged interfaces, depending on the ratio between dye and interface. Non polar solvent can reduce photosensitizer aggregation and increase the monomer-to-dimer ratio. Earlier studies conducted by Severino et al. (Photochem Photobiol. 77(5), pp.459-68, 2003) have shown that the photophysical and photochemical characteristics of MB were modulated by the amount and the type of dimer formed. Dimer formation may decrease the production of singlet oxygen and therefore a formulation that stabilizes monomer is preferred for therapeutic purpose. In particular, increased aggregated MB reduces the formation singlet oxygen yield and by that reduces the efficacy of the MB in the formula to have a photosensitizer effect. Despite its short half-life time (~3.5 $\mu$s in water), singlet oxygen exerts strong cytotoxic effects, destroying cellular constituents, such as organelles, proteins, nucleic acids, cholesterol etc. In non-polar solvents similar to cell membranes, singlet oxygen has a considerably longer half-life (in ethanol approx. 20 light seconds, in chloroform approx. 250 light seconds). Neither the sensitizing dye nor light alone can induce the cytotoxic effect, however the two together are capable of creating ROS which is effective at killing microbes. Stabilization of the photosensitizer can be achieved using of a combination of a nonpolar and polar solvent. A preferable mixture of buffer (polar) and other components such as ethanol and glycerin is one that is sufficiently non-polar such that it is capable of stabilizing the photosensitizer and also dissolve it.

Light Source

The light source selected for photodynamic therapy should emit at a wavelength corresponding to the absorption or excitation of the photosensitizer to have the greatest effect. The light source can be incorporated into a dedicated apparatus for phototherapy treatment. In particular, the light source administers energy to the body which, in the presence of photosensitizer, results in breakdown of microbes. The photosensitizer excitation wavelength may vary depending upon the particular photosensitizing compound used and the light intensity. The waveband of absorption of the photosensitizer should also preferably be between 600 and 800 nm in order to avoid skin phototoxicity. This therapeutic window minimizes absorption during exposure to typical daylight (wavelength 400-600 nm), and minimizes light absorption by water molecules, which increases at wavelengths above 800 nm. (Plaetzer et al., Lasers Med. Sci. 24, pp.259-268, 2009; Sekkat et al., Molecules, 17, pp.98-144, 2012) The light source is optionally an LED (light emitting diode) light source. Both coherent (lasers) and non-coherent (diode emission of light - LED and lamps) light sources can be used for PDT (Nyman and Hynninen, J. Photochem. Photobiol. 8 Biol. 73, pp.1-28, 2004). In an example, chlorophyll a has an absorption band in the range from 600-700 nm and chlorophyll b has a absorption band in the range from 600-675 nm. The light source can also vary in energy density based on photosensitizer dose and the optimal combination of all factors in order to cause delayed growth or death of the fungi and to improve the therapeutic outcome. Lasers are able to deliver light with high degrees of monochromaticity that can be focused into an optic fiber. However, the high cost and the difficulties to transport are some of the drawbacks for the use of lasers in PDT. LEDs are less expensive, easily transportable and, with the discovery of photosensitizers with longer wavelengths, are increasingly being used in experimental and clinical applications of PDT (Hamblin et al., Cancer Res. 56, pp.5205-5210, 1996; Nyman and Hynninen, J. Photochem. Photobiol. 8 Biol. 73, pp.1-28, 2004). For white or fluorescent lamps, minimizing ultra-violet emissions is important to avoid mutagenesis, as well as infrared, to minimize the risk of heating host tissues (Donnelly et al., Microbiol. Res. 163, pp.1-12, 2008). Additionally, it has been found that red light penetrates ~3-4 nm whereas blue light penetrates ~1.5 nm (Donnelly et al., Microbiol. Res. 163, pp.1-12, 2008; Garland et al., Future Med. Chem. 1, pp.667-691, 2009). For the methylene blue activation, monochromatic red light is preferred. For other photosensitizers the selection of monochromatic wavelength for the treatment light depends on the max absorption peak or the photosensitizer.

In addition to the first wavelength of light for the purpose of activating the photosensitizer, a second wavelength may also be used in order to speed up the healing process, increase proliferation of fibroblasts, increase the amount elastin and collagen in the affected area, and reduce inflammation. The present PDT can therefore contribute to the overall healing process. In one example, in a nail treatment, part of the problem is that the nailbed gets inflamed and as a result of the infection the nail growth is very slow. Low-level laser (light) therapy (LLLT) is a fast-growing technology used to treat a multitude of conditions that require stimulation of healing, relief of pain and inflammation, and restoration of function. Although the skin is the organ that is naturally exposed to light more than any other organ, it still responds well to red and near-infrared (NIR) wavelengths. The photons are absorbed by mitochondrial chromophores in skin cells. Consequently electron transport, adenosine triphosphate (ATP) nitric oxide release, blood flow, reactive oxygen species increase and diverse signaling pathways get activated from light excitation. Stem cells can also be activated allowing increased tissue repair and healing. In dermatology, LLLT has shown to have beneficial effects on wrinkles, acne scars, hypertrophic scars, and healing of burns. Inflammatory diseases such as psoriasis and acne can also benefit. The non-invasive nature and almost complete absence of side-effects encourages further testing in dermatology. Four colors are preferable: NIR, red, blue and green, in a preferable range of 440-1100nm, using either low level lasers or LEDs. While all colors have been found to be effective at boosting the cells ability to heal, green light has been found to be very effective at wound healing in particular. Accordingly, using an additional light wavelengths simultaneously with the wavelength required to activate the photosensitizer can have other benefits such as to enhance healing at the site of microbial infection. An apparatus can be configured for delivering light of the desired wavelength or wavelengths. The apparatus can have a single light source or lamp which emits at the desired wavelength, two or more light sources which each emit at a desired wavelength, or one or more light sources that emits at multiple wavelengths that include at least one of the desired wavelengths.

One problem with treatment of infected nails is the resulting slow growth of the infected nails. Using at least two ranges of wavelengths can result in improved wound healing and promote tissue repair concurrently with treatment for the microbial infection. In nail infections, the combination of red and blue light, for example, is one preferable combination, with red wavelength (e.g. around 630 nm) effective at activating the photosensitizer and blue (e.g. around 405 nm) helping the healing process of the epidermis underneath the nail. The light therapy can thereby be used to reduce the inflammation in the skin, increase local blood circulation, and promote faster healing which will contributes to a faster growth of the nail.

Composition

The shelf-life of the presently described composition provides a variety of treatment options for patients, which is useful in the treatment of topical microbial infections. For onychomycosis a stable composition is especially useful as the requirement for treatment is generally many weeks to months, and the prolonged shelf life without requirement for special storage provides improved efficacy of a stored solution. The vehicle composition can comprise an aqueous component, an organic solvent-based component, or a combination of aqueous and organic or solvent-based components. A multiphase composition may encompass both aqueous and organic phases. The organic or solvent component can be polar or non-polar, and the composition can further comprise one or more emulsifiers or additional solvents or components. In one embodiment the composition comprises glycerin in 1-50 v/v%. isopropanol, ethanol, or a mixture thereof in 5-90 v/v%, and water in 10-90 v/v%. Preferably, the vehicle comprises glycerin in 10-50 v/v%, isopropanol or ethanol or a mixture thereof in 5-35 v/v%, and a buffer in 25-75 v/v%. In another embodiment, the amount of ethanol and/or isopropanol in the composition can vary from 10%-90%, and the ethanol can be replaced with another suitable alcohol or pharmaceutically acceptable solvent. The amount of photosensitizer can vary from 0.01%-10% in the composition. One preferred photosensitizer is methylene blue. The amount of *inula viscosa* extract can vary in the composition from, for example, 0.1%-10%. The amount of water can vary from 10%-90%, and can be in the form of buffer. In one embodiment the buffer is PBS buffer. The amount of glycerin in the composition can vary from 1%-50%. An emulsifier may also be added to the composition in an amount of, for example, between 0%-5%. According to testing at the clinic it has found that these vehicles allow for better penetration into the nail, better spread on the nail, and is generally easier to work with, in comparison to water or buffer only due to the composition's higher viscosity and lower surface tension.

Other components can be added to the vehicle composition to provide additional stability, handling, shelf-life, or other advantageous properties. It is understood that any composition or vehicle components should be chemically compatible with the other components of the composition as well as safe for topical use. Chelators are ingredients that complex with and inactivate metallic ions to prevent their adverse effects or undesirable chemical reactions with composition components. Common chelating agents include ethylenediaminetetraacetic acid (EDTA) and its derivatives, etidronic acid and its derivatives, galactaric acid, sodium metasilicate and phosphate derivatives. Disodium EDTA and tetrasodium EDTA are two popular chelators used in the personal care industry to increase effectiveness and improve stability of compositions. The vehicle composition can also comprise one or more surfactants, which serve to reduce the surface tension of the composition and by that allow better penetration of the active species into the skin, mucous membrane or nail. Surfactants can also encourage more spreading on nails and can also be less messy in comparison to water or buffer alone. Surfactants can also act to modify the porosity of hydrated pores of a nail plate, thereby improving the permeability of the composition through the nail plate. Non-limiting examples of surfactants include sodium laureth sulfate (SLS), polysorbate 20 (Tween 20™) , PEG 40, and poloxamer.

Penetration enhancers are compounds in a topical or dermatological vehicle or preparation which penetrate into skin to reversibly decrease the barrier resistance of penetration of active species through the skin. Penetration enhancers can be particularly useful particularly in nail preparations to assist penetration of the photosensitizer and antimicrobial through the dense keratin to the nail bed to the site of infection. Non-limiting examples of penetration enhancers include sulphoxides such as dimethylsulphoxide (DMSO), azones such as laurocapram, pyrrolidones such as 2-pyrrolidone, alcohols such as ethanol and isopropanol, alkanols such as decanol, glycols such as propylene glycol-PEG such as PEG-6, PEG-8, PEG-100, and other polyethylene glycols, as well as nano particles and terpenes. Viscosity enhancers can also be added to the composition to make the composition easier to apply and sufficiently thick so as to remain on the skin, nail and/or nail bed during penetration. Non-limiting examples of viscosity enhancers include acacia, tragacanth, alginic acid, carrageenan, locust bean gum, guar gum, gelatin, methylcellulose, sodium carboxymethylcellulose, Glycerin, Hyaluronic acid, wax and polywax (Cetearyl Alcohol, PEG-150 Stearate, Polysorbate 60, Hydrogels and Steareth-20. It mirrors the properties of Cetyl Alcohol) and carbopol®. Suitable formulation of the vehicle solution assists penetration of the active ingredients through the nail or skin effectively.

The pH of the composition can also be adjusted based on the chemical properties of the components in the composition, and the aqueous component may further be buffered. For methylene blue, for example, spectroscopic data and the protonation constant of 3MBÞ (pKa = 7.2) suggest that at basic pH, the type II photodynamic therapy mechanism is favored, while at low pH, the protonation rate of the central ring nitrogen is higher than the reaction with oxygen, and the type I photodynamic therapy mechanism dominates resulting in radicals. MB is thought to bind strongly to the walls of the fungus, and it is suggested that at pH lower than 6, the major transient species is 3MBH2Þ, which forms less singlet oxygen than the higher pH 3MBÞ triplet. Therefore, assuming that these are the major reactive species that cause the inactivation of fungus, the most effective fungus inactivation by MB is achieved by weakly buffered solutions at pH 7.5 and above, such as pH 7.5 - 9. (Chen et al., J Phys Chem A. 115(13): pp.2702-7, 2011 Apr 7) Combination therapy is a common approach to reduce antimicrobial resistance in patients and clinics. The formulation will consist also a solvent in which the photosensitizer is dissolved, such as isopropanol, DMSO, water, acetone, ethanol, or other suitable solvent.

Optionally the vehicle composition can further comprise additional antifungal compounds such as but not limited polyenes, allylamines, imidazoles, triazoles, tolnaftate, ciclopirox, morpholines, griseofulvin, and a combination thereof. Specific examples of synthetic antifungals include bifonazole, clotrimazole, econazole, ketoconazole, miconazole, tioconazole, nystatin, ciclopirox, benzoic acid, terbinafine, tolciclate, and tolnaftate.

Other active ingredients that are not photosensitizers can also be added to the present formula to provide additional pharmaceutical activity to treat infection of the nail and kill dermatophytes. Anti-inflammatory agents can also be added to the composition to reduce the inflammation and contribute to the overall healing. Non-limiting examples of classes of anti-inflammatory agents include nonsteroidal anti-inflammatory drugs and glucocorticoids.

Therapy

To treat a patient with onychomycosis, the nail is first treated with a composition comprising a naturally-derived antimicrobial, a photosensitizer, formulated in a pharmaceutically acceptable vehicle. The composition is then allowed sufficient penetration time such that it can be absorbed or moved to the site of infection. Once the composition has penetrated the nail to the site of infection, the site is irradiated with a light source at a wavelength absorbed by the photosensitizer. Upon light excitation of the photosensitizer, reactive oxygen species are generated to destroy microbes at the site of infection. Since light can penetrate through the nail, the irradiating step can be achieved by irradiating either directly on the locus (with nail removed), or indirectly through the nail with a light source at a wavelength absorbed by the photosensitizer so as to destroy microbes at the locus of infection. The area can further be treated with a second wavelength of light known to be efficacious in skin therapy, tissue repair, and/or wound healing. An apparatus for photodynamic therapy can also be used that provides a first light source for emitting light at a first wavelength in a waveband of absorption of a photosensitizer and a second light source for emitting light at a second wavelength for promoting tissue repair, wound healing, or a combination thereof. The second wavelength can be, for example, in the range of near infrared, red, blue, and green.

The present therapy can be practiced in a variety of locations, including but not limited to practitioners such as foot care nurses, podiatrists, chiropodists, aestheticians, and dermatologists. End users-take home kit can also be provided to patients for home care.

Iontophoresis can be used to increase skin permeation of the active ingredients is the method where the movements of ions across a membrane enhanced using an externally applied potential difference. The principle barrier to the transport of positively charged photosensitizers, for example, such as methylene blue or other charged photosensitizer into an across the skin is through the stratum corneum (SC). In iontophoresis, cationic or neutral active substances can be placed under an anode, and anionic therapeutic agents can be placed under a cathode. When a low voltage and low current density is used through the charged device, the correspondingly charged photosensitizer can move more readily through the skin. In particular, water soluble photosensitizers that are charged at composition pH can be positively influenced by iontophoresis, and guided through the skin by the applied opposite charged device. Charged photosensitizers include but are limited to methylene blue, rose bengal, acridine, curcumin, hypericin, toluidine blue, and 5-amino levulinic acid (ALA). The pH of the solution may be further adjusted to shift the photosensitizer into its charged state prior to skin application to potentially improve the iontophoresis efficacy.

Decolourizing solution

Subsequent to treatment of the nail with coloured photosensitizer a decolourizing solution or formulation can be used to remove the colour from the nail. The decolourization or colour neutralization of methylene blue +, for example, can be obtained by CSN- (partial), $H_2O_2$, Ultraviolet light, ascorbic acid, strong oxidant as Ce(IV), -OH or $H_2O_2$ in basic medium, sulfide ($Na_2S$), and other reducing sugars and reducing agents, used alone or in combination. Neutralization or decolourizing of the blue colour of the methylene blue, for example, can occur on the nail using aqueous solution of alkaline and reduction agent such as glucose or dextrose. Additional reducing agents that can be used to decolour coloured photosensitizers applied to the skin as part of the treatment composition include hydrogen peroxide ($H_2O_2$), formic acid (HCOOH), ascorbic acid ($C_6H_8O_6$), carbon monoxide (CO) or $CO_2$, and other reducing agents. In one example, a neutralizing solution is prepared from glucose-3% and KOH-2.7%, with a pH at the range of about 8-10.

EXAMPLES

Example 1: Appropriate carrier composition - *In vitro*

The carrier is consisting of three components, glycerol, ethanol and water as the solvent. For glycerol, six volume percentage is chosen: 60%, 30%, 15%, 7.5%, 3.75% and 0; For ethanol, six volume percentage is chosen: 40%, 20%, 10%, 5%, 2.5% and 0; For water, its volume percentage plus the glycerol and ethanol volume percentage was 100% for every combination. In this experiment, the volume ratio of *T.rubrum* and 2X RPMI medium was 1:19. The compounds were added to wells of 96-well microtiter plate to measure the OD value in order to determine growth. Results are shown in Table 1. Glycerine is preferred to be at 30% in term of providing the best surface tension and viscosity of the composition for nail application. Based on the results below and the requirements above, and in order to have an added value of antimicrobial effect, the optimized ethanol concentration is at a range of 10%-40%. Glycerine may have beneficial effect on the fungus growth and can be replaced with propylene glycol.

Table 1:

|  |  | Glycerin | | | | | |
|---|---|---|---|---|---|---|---|
|  |  | 0 | 3.75% | 7.50% | 15% | 30% | 60% |
| Ethanol | 0 | N | N | N | N | √ | √ |
|  | 2.50% | N | N | N | N | √ | × |
|  | 5% | N | N | N | N | √ | × |
|  | 10% | × | × | × | × | × | × |
|  | 20% | × | × | × | × | × | × |
| √: Fungal growth is inhibited but not killed<br>X: No fungal growth and all cells are killed<br>N: Cells are growing, not inhibited nor killed | | | | | | | |

Example 2: Use of Methylene Blue in phototherapy - *Ex vivo*

Sterilized nail pieces were placed on fresh *T.rubrum* lawn to be infected for at least 10 days with dorsal side upwards. Nail pieces were treated with Methylene blue (MB) in a carrier in range of concentrations vary from 0.03125% - 0.25%. Control was carrier solution with no Methylene blue. On a separate experiment, MB alone and light alone did not kill nor inhibit the fungus growth. The nails were exposed to red light (640nm, 9W - 3 LED lights, 3W each) for 30min. Amount of viable fungal colonies of *Trichophyton rubrum* after treatment relative to control was observed using microscope and once visual continued to be monitored with the naked eye. Results are shown in Table 2, where "G" indicates growth and "N" indicates no growth. As shown, after 3 days the control showed fungal growth. After 5 days mycelium growth was found for the 0.0625% and 0.03125% treatments. After 6 days of the treatment mycelium was found for the 0.125% treatments. After 12 days of the treatment, 0.25% MB showed growth. Meaning that there is a need for a secondary treatment or alternative treatment in order to obtain prolonged inhibition of re-growth.

Table 2:

|  | Day 3 | Day 4 | Day 6 | Day 7 | Day 8 | Day 9 | Day 10 | Day 12 | Day 13 |
|---|---|---|---|---|---|---|---|---|---|
| Control | G | G | G | G | G | G | G | G | G |
| 0.03% | N | N | G | G | G | G | G | G | G |
| 0.0625% MB | N | N | G | G | G | G | G | G | G |
| 0.125% MB | N | N | N | G | G | G | G | G | G |
| 0.25% MB | N | N | N | N | N | N | N | N | G |

Example 3: Use of *Inula Viscosa* as an antimicrobial for treatment of Onychomycosis - *In vitro*

The use of *Inula Viscosa* was investigated as a long term treatment solution for onychomycosis. It was shown that secondary treatment of onychomycosis with *Inula Viscosa* after primary treatment with photodynamic therapy at a wavelength of 640nm demonstrates prolonged inhibition of fungal re-growth.

5 - 6mm *T. rubrum* cubes were cut from a lawn to test the long term effect of the *inula viscosa*. Cubes were submerged in the carrier plus 1% inula for 5-10min, washed with water and observed daily for growth (triplicates). The wash was done to resemble a treatment where a person gets the treatment and washes off the compound later on, simulating periodic washing or showering. Amount of viable fungal colonies of *Trichophyton rubrum* after treatment relative to control was observed using microscope and once visual continued to be monitored with the naked eye. Results indicated that *Inula viscosa* is active for at least 15 days after application and that application of a composition comprising *Inula viscosa* further contributes to inhibiting growth of fungal cells that survived the photodynamic light therapy with the photosensitizer, as shown in Table 3, with amount of growth graded from 0 (no growth) to 3 (significant growth).

Table 3:

|  | Day 1 | Day 2 | Day 3 | Day 4 | Day 5 | Day 6 | Day 7 | Day 8 | Day 9-15 |
|---|---|---|---|---|---|---|---|---|---|
| **Control-not treated** | 1,1,1 | 2,2,2 | 2,2,2 | 3,3,3 | 3,3,3 | 3,3,3 | 3,3,3 | 3,3,3 | 3,3,3 |
| **Control-wash 5min** | 0,0,0 | 0,0,0 | 2,2,0 | 3,3,2 | 3,3,2 | 3,3,3 | 3,3,3 | 3,3,3 | 3,3,3 |
| **Control-wash 10min** | 0,0,0 | 0,0,0 | 0,0,0 | 2,2,0 | 2,3,0 | 3,3,2 | 3,3,2 | 3,3,3 | 3,3,3 |
| **Inula-5min** | 0,0,0 | 0,0,0 | 0,0,0 | 0,0,0 | 0,0,0 | 1,0,0 | 2,0,0 | 3,2,2 | 3,3,3 |
| **Inula-10min** | 0,0,0 | 0,0,0 | 0,0,0 | 0,0,0 | 0,0,0 | 0,0,0 | 0,0,0 | 0,0,0 | 0,0,0 |

Example 4: *Inula viscosa* has a phototherapeutic effect - *Ex vivo*

In the absence of an additional photosensitizer, it has been found that inula viscosa can serve as a photosensitizer and have a phototherapeutic effect. Without being bound by theory, it is hypothesized that this effect is perhaps due to inula stabilization of chlorophylls, and/or the unique chlorophylls found in *inula viscosa* leaves that are capable of acting as photosensitizers in the presence of light therapy.

Sterilized nail pieces were placed on fresh *T.rubrum* lawn to be infected for at least 10 days with dorsal side upwards. Nail pieces were then soaked in 1% Inula composition in carrier, for 5 or 10 min and then exposed to light for 30 min. The samples were then tested with and without light exposure at a wavelength of 640 nm and compared to control samples that were washed after 5 or 10 min. Amount of viable fungal colonies of *Trichophyton rubrum* after treatment relative to control was observed using microscope and once visual continued to be monitored with the naked eye. The results are observed in Table 4, where "G" indicates growth and "N" indicates no growth.

Table 4:

|  | Treatment | Day1 | Day2 | Day3 | Day4 | Day6 |
|---|---|---|---|---|---|---|
| **Control** | Water 5min-No light | G | G | G | G | G |
|  | Water 10min-No light | G | G | G | G | G |
|  | Carrier, 5min no light | G | G | G | G | G |
|  | Carrier, 10min no light | G | G | G | G | G |

(continued)

| | Treatment | Day1 | Day2 | Day3 | Day4 | Day6 |
|---|---|---|---|---|---|---|
| **Treatment-NO light exposure** | Inula-5min no light | N | G | G | G | G |
| | Inula-10min no light | N | N | G | G | G |
| **Treatment-WITH light exposure** | Inula-5min light | N | N | G | G | G |
| | Inula-10min light | N | N | N | N | N |

This experiment showed that *Inula viscosa* can potentially serve as a natural photosensitizer. In addition, with dual activity as photoactivation and antifungal an efficacy increase is apparent as compared activity with no light, demonstrating that *inula viscosa* can serve as a photosentisizer on its own without an additional photosensitizer.

Example 5: Photosensitizer Composition Preparation and Use

A composition was prepared comprising, in two separate phases with the following ingredients: methylene blue (0.25%), *inula viscosa* extract (1%), water (48.25%), ethanol (20%), glycerin (30%) and non-ionic emulsifier (Lipowax D, 0.5%). An aqueous phase was prepared by mixing methylene blue as an additional photosensitizer into a mixture of the deionized water and ethanol. For reference, a similar composition can be prepared without an additional photosensitizer. The hydrophobic phase was prepared by mixing non-ionic emulsifier such as Lipowax D, *Inula viscosa* and glycerin. The hydrophobic phase was then added to the aqueous phase while mixing vigorously until a stable emulsion was formed. The emulsion was kept at room temperature until use.

Example 6: Exemplary protocol for treatment of onychomycosis

Onychomycosis has been treated in a number of patients using the following protocol. Data was collected for the treatment and patient effects have been positive and effectiveness of the treatment as it requires a minimal number of visit to the clinic and as such does not have issues with compliance due to lengthy treatments. This protocol and the described compositions and methods are capable of treating toenail fungus quickly, effectively, and safely. The compositions and methods of the present invention described herein satisfy these and other needs. An exemplary protocol for the therapy for treatment of onychomycosis is as follows:

1. File the dorsal side of the nail/s gently

2. Apply the antifungal composition of the present invention to the nail and wait for 10min to allow it to penetrate.

3. Irradiating the nail/s with a light source at a wavelength absorbed by the photosensitizer of the antifungal composition so as to destroy microbes.

Optionally, additional antifungal agent in a secondary treatment can be applied after the phototherapy, which can be additional application of *Inula Viscosa* or another antifungal agent. The secondary treatment composition should be formulated in a pharmaceutically acceptable delivery system, and can optionally comprise at least one pharmaceutically acceptable nail penetration enhancer, emulsifier, or both. Secondary treatment step can be done once, every day, or multiple times a day after primary photodynamic treatment until the next light irritation or as a maintenance therapy. It can be also used as prevention treatment of elimination of recurrence. This protocol can be repeated at two-week intervals over a predetermined treatment period as needed (e.g. weeks, or months) until reduction or arresting of growth is acceptable.

Therapy of the present invention may also optionally include (i) reducing the thickness of the nail and/or (ii) creating micro-channels from top of the nail to bottom of the nail. It is preferred to reduce the thickness prior to the treatment that the desired thickness is a thickness that causes little or no discomfort to the patient. Propylene glycol can also optionally be used after the treatment as it has hygroscopic activity and can further assist in killing any fungus cells that did survive the phototherapy treatment. In one additional option, it has been further found that subsequent to phototherapy, treatment of the nail with propylene glycol can dry out the fungal cells further and positively contribute to prolongation of the treatment efficacy.

**Claims**

1. A photosensitizer composition, comprising:

an *Inula viscosa* extract acting as a photosensitizer; and
a pharmaceutically acceptable vehicle for enhancing spread of the composition on a nail and penetration of the composition into the nail, the pharmaceutically acceptable vehicle comprising:

glycerin or propylene glycol in 1-50 v/v%;
isopropanol, ethanol, or a mixture thereof in 5-90 v/v%;
water in 10-90 v/v%; and
an emulsifier in 0.5-5 v/v %.

2. The photosensitizer composition of claim 1, further comprising one or more additional photosensitizer, penetration enhancer, preservative, chelator, synthetic antifungal, or anti-inflammatory compound.

3. The photosensitizer composition of claim 2, wherein the additional photosensitizer is one of hypericin, a chlorophyll, curcumin, methylene blue, a porphyrin, a phthalocyanine, a phenothiazinium, a benzoporphyrin, a haematoporphyrin, a pyrrole, a tetrapyrrolic compound, a pyrrolic macrocycle, a porfimer and 5-aminolevulinic acid.

4. The photosensitizer composition of any one of claims 1-3, wherein the composition further comprises an additional naturally-derived antimicrobial selected from the group consisting of a cinnamodial sterol, furanone, quinine, resorcinol, or terpenoid.

5. The photosensitizer composition of any one of claims 1-4, wherein the *Inula viscosa* extract is present in the composition in 0.1%-10% v/v.


**Patentansprüche**

1. Photosensibilisator-Zusammensetzung, die aufweist:

einen *Inula viscosa* Extrakt, der als Photosensibilisator fungiert; und
einen pharmazeutisch akzeptierbaren Trägerstoff zum Verbessern des Verbreitens der Zusammensetzung auf einem Nagel und des Eindringens der Zusammensetzung in den Nagel, wobei der pharmazeutisch akzeptierbare Trägerstoff aufweist:

Glyzerin oder Propylenglykol in 1-50 Vol.-%;
Isopropanol, Ethanol oder eine Mischung davon in 5-90 Vol.-%;
Wasser in 10-90 Vol.-%; und
einen Emulgator in 0,5-5 Vol.-%.

2. Photosensibilisator-Zusammensetzung nach Anspruch 1, die des Weiteren einen oder mehrere zusätzliche Photosensibilisatoren, Penetrationsverbesserer, Konservierungsmittel, Chelator, synthetisches Antimykotikum, oder entzündungshemmende Zusammensetzung aufweist.

3. Photosensibilisator-Zusammensetzung nach Anspruch 2, wobei der zusätzliche Photosensibilisator eines ist von einem Hypericin, einem Chlorophyll, Curcumin, Methylenblau, einem Porphyrin, einem Phthalocyanin, einem Phenothiazin, einem Benzoporphyrin, einem Haematoporphyrin, einem Pyrrol, einer Tetrapyrrolverbindung, einem Pyrrol-Makrozyklus, einem Porfimer und 5-Aminolävulinsäure.

4. Photosensibilisator-Zusammensetzung nach einem der Ansprüche 1-3, wobei die Zusammensetzung des Weiteren eine zusätzliche natürlich abgeleitete antimikrobielle Substanz aufweist, die ausgewählt wird aus der Gruppe bestehend aus einem Cinnamodial-Sterol, Furanon, Chinin, Resorcinol oder Terpenoid.

5. Photosensibilisator-Zusammensetzung nach einem der Ansprüche 1-4, wobei der *Inula viscosa* Extrakt in der Zusammensetzung in 0,1 - 10 Vol.-% vorliegt.

## EP 3 765 081 B1

**Revendications**

1. Composition de photosensibilisant, comprenant :

   un extrait de *Inula viscosa* agissant comme photosensibilisant ; et
   un véhicule pharmaceutiquement acceptable pour améliorer l'étalement de la composition sur un ongle et la
   pénétration de la composition dans l'ongle, le véhicule pharmaceutiquement acceptable comprenant :

   de la glycérine ou du propylène glycol à raison de 1 à 50 % v/v ;
   de l'isopropanol, de l'éthanol ou un mélange de ceux-ci à raison de 5 à 90 % v/v ;
   de l'eau à raison de 10 à 90 % v/v ; et
   un émulsifiant à raison de 0,5 à 5 % v/v.

2. Composition de photosensibilisant selon la revendication 1, comprenant en outre un ou plusieurs photosensibilisants
   additionnels, composé activateur de pénétration, conservateur, chélatant, antifongique synthétique ou anti-inflammatoire.

3. Composition de photosensibilisant selon la revendication 2, dans laquelle le photosensibilisant supplémentaire est
   un parmi : l'hypericine, une chlorophylle, la curcumine, le bleu de méthylène, une porphyrine, une phtalocyanine,
   un phénothiazinium, une benzoporphyrine, une hématoporphyrine, un pyrrole, un composé tétrapyrrolique, un
   macrocycle pyrrolique, un porfimère et l'acide 5-aminolévulinique.

4. Composition de photosensibilisant selon l'une quelconque des revendications 1 à 3, dans laquelle la composition
   comprend en outre un antimicrobien d'origine naturelle supplémentaire choisi dans le groupe constitué par un stérol
   de cinnamodial, une furanone, une quinine, un résorcinol ou un terpénoïde.

5. Composition de photosensibilisant selon l'une quelconque des revendications 1 à 4, dans laquelle l'extrait de *Inula
   viscosa* est présent dans la composition à raison de 0,1 % à 10 % v/v.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090233914 A1
- US 20090234270 A1
- WO 2012090194 A2

### Non-patent literature cited in the description

- **HARRIS ; PIERPOINT.** *Med. Res. Rev.,* 2012, vol. 32, 1292-1327
- **CALZAVARA-PINTON et al.** *J. Photochem. Photobiol. 8 Biol.,* 2012, vol. 78, 1-6
- **SEKKAT et al.** *Molecules,* 2012, vol. 17, 98-144
- **MANTAREVA et al.** *Eur. J. Med. Chem.,* 2011, vol. 46, 4430-4440
- **CORMICK et al.** *Eur. J. Med. Chem.,* 2009, vol. 44, 1592-1599
- **CHITRODA et al.** *International Journal of Scientific Study,* August 2014, vol. 2, 5
- **SYTAR et al.** *Pharmaceutical Biology,* 2016, vol. 54, 12
- **KASHEF et al.** *Photodiagnosis Photodyn Ther.,* 2013, vol. 10, 150-155
- **ÇIRAK et al.** *Plant Protect Sci.,* 2005, vol. 41, 109-114
- **FENNER et al.** *Phytomedicine,* 2005, vol. 12 (3), 236-40
- **PRASAD, S. et al.** *Cancer Res Treat.,* January 2014, vol. 46 (1), 2-18
- **DOVIGO et al.** *Photochem. Photobiol.,* 2013, vol. 87, 895-903
- **SHARMA et al.** *Biosci. Rep.,* 2010, vol. 30, 391-404
- **BERHAIL BOUDOUDA H.** *Der Pharmacia Lettre,* 2012, vol. 4 (6), 1863-1867
- **SAWSAM et al.** *European Journal of Medicinal Plants,* 2013, vol. 3 (3), 394-404
- **MAOZ M ; NEEMAN, J.** *Ethnopharmacol,* August 2000, vol. 71 (3), 479-82
- **SEVERINO et al.** *Photochem Photobiol.,* 2003, vol. 77 (5), 459-68
- **PLAETZER et al.** *Lasers Med. Sci.,* 2009, vol. 24, 259-268
- **NYMAN ; HYNNINEN.** *J. Photochem. Photobiol. 8 Biol.,* 2004, vol. 73, 1-28
- **HAMBLIN et al.** *Cancer Res.,* 1996, vol. 56, 5205-5210
- **NYMAN ; HYNNINEN.** *. Photochem. Photobiol. 8 Biol.,* 2004, vol. 73, 1-28
- **DONNELLY et al.** *Microbiol. Res.,* 2008, vol. 163, 1-12
- **GARLAND et al.** *Future Med. Chem.,* 2009, vol. 1, 667-691
- **CHEN et al.** *J Phys Chem A.,* 07 April 2011, vol. 115 (13), 2702-7